# EUROPÄISCHE PATENTANMELDUNG

(11) **EP 4 850 719 A1**
(43) Veröffentlichungstag der Anmeldung: **09.12.2026**
(21) Anmeldenummer: 26182557.4
(22) Anmeldetag: 02.06.2026
(51) Int. Cl.: A61M 5/168

(54) **MEDIZINISCHE VORRICHTUNG ZUM ÜBERWACHEN EINES FLUIDDRUCKS IN EINER INJEKTIONSLEITUNG**

(30) Priorität: 04.06.2025 DE 102025121748
(71) Anmelder: B. Braun Melsungen AG, 34212 Melsungen (DE)
(72) Erfinder: Klotzbach, Roman, 34225 Baunatal (DE)
(74) Vertreter: Patentanwälte Ruff, Wilhelm, Beier, Dauster & Partner mbB

(57) **Zusammenfassung**

Die Erfindung betrifft eine medizinische Vorrichtung zum Überwachen eines Fluiddrucks in einer Injektionsleitung, aufweisend einen Dehnungsmessstreifen, der zum Erfassen einer durch den Fluiddruck verursachten elastischen Deformation der Injektionsleitung und zum Ausgeben eines Messsignals eingerichtet ist, das den erfassten Fluiddruck repräsentiert, eine Auswerteeinrichtung, die mit dem Dehnungsmessstreifen verbunden ist, wobei die Auswerteeinrichtung eingerichtet ist zum Vergleichen des Messsignals mit wenigstens einem vorgegebenen Schwellenwert, und zum Erzeugen wenigstens eines Überwachungssignals in Abhängigkeit des Vergleichs, sowie eine Ausgabeeinrichtung und/oder eine Übertragungseinrichtung, wobei die Ausgabeeinrichtung mit der Auswerteeinrichtung verbunden und zum Ausgeben des wenigstens einen Überwachungssignals in einer für einen Benutzer wahrnehmbaren Form eingerichtet ist, wobei die Übertragungseinrichtung mit der Auswerteeinrichtung verbunden und zum Übertragen des wenigstens einen Überwachungssignals an eine externe Empfangseinrichtung eingerichtet ist. Die Erfindung betrifft zudem ein Verfahren zum Überwachen eines Fluiddrucks in einer Injektionsleitung.

## Beschreibung

Die Erfindung betrifft eine medizinische Vorrichtung zum Überwachen eines Fluiddrucks in einer Injektionsleitung.

Medizinische Eingriffe sind oftmals nicht ohne eine Anästhesie durchführbar, wobei grundsätzlich zwischen der Allgemeinanästhesie und der Lokalanästhesie unterschieden werden kann. Einen bedeutsamen Teil der Lokalanästhesie stellt die sogenannte Regionalanästhesie dar, insbesondere die periphere Regionalanästhesie.

In der peripheren Regionalanästhesie wird das Anästhetikum in unmittelbarer Nähe des zu betäubenden Nervs verabreicht. Dabei ist der zur Injektion des Anästhetikums verwendete Fluiddruck von besonderer Bedeutung. Zu hohe Fluiddrücke können zu einer Schädigung des Nervs führen.

Aus der WO 2003/101526 A1 ist eine medizinische Vorrichtung zum visuellen Darstellen des Fluiddrucks in einer Injektionsleitung bekannt. Die bekannte Vorrichtung weist einen Anzeigestift auf, der in Abhängigkeit des anliegenden Fluiddrucks unterschiedlich weit aus einem Gehäuse der Vorrichtung herausgeschoben wird. Über eine an dem Anzeigestift angebrachte Farbcodierung kann der Benutzer den Fluiddruck visuell überwachen.

Aus der DE 10 2020 210 756 A1 ist eine weitere medizinische Vorrichtung zum Überwachen eines Fluiddrucks in einer Injektionsleitung bekannt. Die bekannte Vorrichtung weist einen Fluidpfad für das zu injizierende Fluid auf, der mit einem piezochromen Material versehen ist, das bei Einwirkung eines Fluiddrucks von < 100 bar eine für das menschliche Auge wahrnehmbare piezochrome Farbänderung zeigt.

Aufgabe der Erfindung ist es, eine medizinische Vorrichtung zum Überwachen eines Fluiddrucks in einer Injektionsleitung bereitzustellen, die Vorteile gegenüber dem Stand der Technik bietet.

Diese Aufgabe wird durch das Bereitstellen einer medizinischen Vorrichtung mit den Merkmalen des Anspruchs 1 gelöst. Vorteilhafte Weiterbildungen sind in den abhängigen Ansprüchen angegeben. Der Wortlaut der Ansprüche wird durch Bezugnahme zum Gegenstand der Beschreibung gemacht.

Die erfindungsgemäße medizinische Vorrichtung dient zum Überwachen eines Fluiddrucks in einer Injektionsleitung und weist einen Dehnungsmessstreifen und eine Auswerteeinrichtung auf. Bei einer erfindungsgemäßen Ausgestaltung weist die medizinische Vorrichtung zudem eine Ausgabeeinrichtung auf. Alternativ oder zusätzlich weist die erfindungsgemäße medizinische Vorrichtung eine Übertragungseinrichtung auf. Der Dehnungsmessstreifen ist zum Erfassen einer durch den Fluiddruck verursachten elastischen Deformation der Injektionsleitung und zum Ausgeben eines Messsignals eingerichtet, das den Fluiddruck repräsentiert. Das besagte Messsignal repräsentiert den Fluiddruck indirekt. Mit anderen Worten: Das Messsignal wird als Funktion der erfassten elastischen Deformation der Injektionsleitung und damit indirekt als Funktion des Fluiddrucks ausgegeben. Die Auswerteeinrichtung ist mit dem Dehnungsmessstreifen verbunden. Die Auswerteeinrichtung ist zum Vergleichen des Messsignals mit wenigstens einem vorgegebenen Schwellenwert und zum Erzeugen wenigstens eines Überwachungssignals in Abhängigkeit des Vergleichs eingerichtet. Die Ausgabeeinrichtung ist mit der Auswerteeinrichtung verbunden und zum Ausgeben des wenigstens einen Überwachungssignals in einer für einen Benutzer wahrnehmbaren Form eingerichtet. Bei einer Ausgestaltung ist die Ausgabeeinrichtung dazu eingerichtet, das wenigstens eine Überwachungssignal in Form eines Lichtsignals auszugeben. Alternativ oder zusätzlich ist die Ausgabeeinrichtung dazu eingerichtet, das wenigstens eine Überwachungssignal in Form eines Tonsignals auszugeben. Die Übertragungseinrichtung ist mit der Auswerteeinrichtung verbunden und zum Übertragen des wenigstens einen Überwachungssignals an eine externe Empfangseinrichtung eingerichtet. Eine Ausgabe und/oder weitergehende Auswertung des mittels der Übertragungseinrichtung übertragenen Überwachungssignals erfolgt in diesem Fall mittels der externen Empfangseinrichtung. Die erfindungsgemäße medizinische Vorrichtung erlaubt eine für den Benutzer einfache sowie zuverlässige Überwachung des Fluiddrucks in der Injektionsleitung. Zudem kann das Überwachungssignal grundsätzlich aufgezeichnet werden, was eine vereinfachte Dokumentation erlaubt. Der Dehnungsmessstreifen ist bei unterschiedlichen Ausgestaltungen unterschiedlich ausgeführt, beispielsweise als Folien-, Draht-und/oder faseroptischer Dehnungsmessstreifen. Grundsätzlich denkbar und möglich sind auch Halbleitervarianten. Das grundsätzliche Messprinzip eines Dehnungsmessstreifens ist jedenfalls im Bereich der Messtechnik allgemein bekannt und basiert auf einer Änderung des elektrischen Widerstands infolge einer Längen- und/oder Querschnittsänderung des Dehnungsmessstreifens. Bei faseroptischen Dehnungsmessstreifen basiert das Messprinzip auf einer Messung eines Interferenzmusters des Lichts. Bei einer bevorzugten Ausgestaltung ist die medizinische Vorrichtung zur mehrfachen Verwendung eingerichtet. In diesem Fall ist der Dehnungsmessstreifen zum Erfassen der elastischen Deformation auf geeignete Weise mit der Injektionsleitung lösbar wirkverbunden. Bei einer weiteren Ausgestaltung ist der Dehnungsmessstreifen in die Injektionsleitung integriert, so dass jedenfalls der Dehnungsmessstreifen der medizinischen Vorrichtung einer Mehrfachverwendung nicht zugänglich ist. Bei der Injektionsleitung handelt es sich vorzugsweise um eine Injektionsleitung zur Verwendung bei einer Regionalanästhesie.

In Ausgestaltung der Erfindung ist die Auswerteeinrichtung dazu eingerichtet, in Abhängigkeit des Vergleichs wenigstens ein erstes Überwachungssignal zu erzeugen, falls der Schwellenwert unterschritten ist, und ein unterschiedliches zweites Überwachungssignal zu erzeugen, falls der Schwellenwert überschritten ist. Bei dieser Ausgestaltung der Erfindung ist die Ausgabeeinrichtung dementsprechend zum Ausgeben wenigstens des ersten Überwachungssignals und des unterschiedlichen zweiten Überwachungssignals eingerichtet. Entsprechendes gilt sinngemäß für die Übertragungseinrichtung, die demnach zum Übertragen wenigstens des ersten Überwachungssignals und des unterschiedlichen zweiten Überwachungssignals eingerichtet ist. Bei dieser Ausgestaltung der Erfindung sind folglich wenigstens zwei Druckbereiche des Fluiddrucks überwachbar. Ein erster Druckbereich liegt vor, wenn der Schwellenwert unterschritten ist. Ein zweiter Druckbereich liegt vor, wenn der Schwellenwert überschritten ist. Demnach ist das erste Überwachungssignal dem besagten ersten Druckbereich zugeordnet und das zweite Überwachungssignal ist dem zweiten Druckbereich zugeordnet. Bei dieser Ausgestaltung der Erfindung kann der Benutzer auf noch weiter vereinfachte Weise den Fluiddruck überwachen. Denn anhand der unterschiedlichen Überwachungssignale ist unmittelbar erkennbar, ob beispielsweise ein niedriger oder hoher Fluiddruck in der Injektionsleitung anliegt. Es versteht sich, dass bei einer entsprechenden Einrichtung der Auswerteeinrichtung auch mehr als die besagten zwei unterschiedlichen Überwachungssignale zur Überwachung zweier unterschiedlicher Druckbereiche erzeugt und ausgegeben werden können. Beispielsweise können ein erstes Überwachungssignal, ein unterschiedliches zweites Überwachungssignal und ein unterschiedliches drittes Überwachungssignal erzeugt und ausgegeben werden, wenn der Fluiddruck in einem ersten Druckbereich, einem unterschiedlichen zweiten Druckbereich bzw. einem unterschiedlichen dritten Druckbereich anliegt. Dies wiederum in Abhängigkeit des besagten Vergleichs des Messsignals mit dem wenigstens einen Schwellenwert.

In weiterer Ausgestaltung der Erfindung weist die Ausgabeeinrichtung wenigstens ein Leuchtmittel auf, das zum Ausgeben des ersten Überwachungssignals und des zweiten Überwachungssignals in Form unterschiedlicher Lichtsignale eingerichtet ist, wobei die unterschiedlichen Lichtsignale sich im Hinblick auf ihre jeweilige Lichtfarbe und/oder Signaldauer unterscheiden. Die unterschiedlichen Lichtsignale sind durch den Benutzer unmittelbar optisch wahrnehmbar, was eine besonders einfache und zuverlässige Überwachung des Fluiddrucks erlaubt. Bei einer bevorzugten Ausgestaltung ist das wenigstens eine Leuchtmittel zum Ausgeben sämtlicher unterschiedlicher Lichtsignale eingerichtet. Bei einer weiteren Ausgestaltung weist die Ausgabeeinrichtung mehrere Leuchtmittel auf, die zur Ausgabe jeweils eines der mehreren unterschiedlichen Lichtsignale eingerichtet sind. Bei einer Ausgestaltung unterscheiden sich die unterschiedlichen Lichtsignale im Hinblick auf ihre jeweilige Lichtfarbe. Beispielsweise können ein grünes, gelbes und/oder rotes Lichtsignal vorgesehen sein. Denkbar und möglichst sind selbstverständlich auch hiervon abweichende Lichtfarben. Bei einer weiteren Ausgestaltung unterscheiden die Lichtsignale sich alternativ oder zusätzlich im Hinblick auf ihre jeweilige Signaldauer. Beispielsweise kann ein zeitlich kurzes Lichtsignal ausgegeben werden, wenn der Fluiddruck in einem ersten niedrigen Druckbereich anliegt. Liegt ein hoher oder zu hoher Fluiddruck an, kann ein Lichtsignal mit durchgängiger Signaldauer ausgegeben werden.

In weiterer Ausgestaltung der Erfindung ist das wenigstens eine Leuchtmittel eine RGB-LED, die zur Ausgabe grüner, gelber und roter Lichtsignale eingerichtet ist, die einen niedrigen, mittleren bzw. hohen Fluiddruck repräsentieren. Dies ist eine bevorzugte Ausgestaltung der Erfindung. Durch die Ausgabe der besagten grünen, gelben und roten Lichtsignale kann der Benutzer auf besonders einfache und zuverlässige Weise erkennen, ob ein niedriger (grünes Lichtsignal), mittlerer (gelbes Lichtsignal) oder hoher Fluiddruck (rotes Lichtsignal) in der Injektionsleitung anliegt. Durch die Ausgabe der unterschiedlichen Lichtsignale mittels ein- und derselben RGB-LED kann ein besonders einfacher, kompakter und damit auch kostengünstiger Aufbau der medizinischen Vorrichtung erreicht werden.

In weiterer Ausgestaltung der Erfindung weist die Ausgabeeinrichtung einen Lautsprecher auf, der zum Ausgeben des ersten Überwachungssignals und des zweiten Überwachungssignals in Form unterschiedlicher Tonsignale eingerichtet ist, wobei die unterschiedlichen Tonsignale sich im Hinblick auf ihre jeweilige Tonhöhe, Tonfolge und/oder Signaldauer unterscheiden. Durch den Lautsprecher und die entsprechende Ausgabe der unterschiedlichen Tonsignale kann der Benutzer den Fluiddruck auf einfache und zuverlässige Weise akustisch, d. h. per Gehör, überwachen. Diese Ausgestaltung der Erfindung erfordert nicht unbedingt einen unmittelbaren Sichtkontakt des Benutzers mit der medizinischen Vorrichtung. Dies im Unterschied zu Ausgestaltungen, die eine optische Ausgabe des wenigstens einen Überwachungssignals vorsehen. Bei einer Ausgestaltung unterscheiden sich die unterschiedlichen Tonsignale im Hinblick auf ihre jeweilige Tonhöhe. Beispielsweise können eine niedrige, mittlere und hohe Tonhöhe einen niedrigen, mittleren bzw. hohen Fluiddruck signalisieren. Bei einer weiteren Ausgestaltung unterscheiden sich die Tonsignale alternativ und zusätzlich im Hinblick auf ihre jeweilige Tonfolge. Beispielsweise kann ein zeitlicher Abstand der Tonfolge und/oder ein Tempo der Tonfolge den Fluiddruck signalisieren. Denkbar und möglich ist beispielsweise, dass eine schnelle Tonfolge einen hohen Druck signalisiert, wohingegen eine langsame Tonfolge einen niedrigen Druck signalisiert. Entsprechende Überlegungen gelten im Hinblick auf eine unterschiedliche Signaldauer der unterschiedlichen Tonsignale. Denkbar und möglich ist auch ein stufenloser Übergang zwischen den unterschiedlichen Tonsignalen oder ein stufenloser An-und/oder Abstieg eines Signals, wodruch gleichsam unterschiedliche Tonsignale erzeugt werden.

In weiterer Ausgestaltung der Erfindung weist die Übertragungseinrichtung eine Funkeinheit auf, die zur drahtlosen Übertragung des wenigstens einen Überwachungssignals eingerichtet ist. Durch die drahtlose Übertragbarkeit des wenigstens einen Überwachungssignals mittels der Funkeinheit kann auf eine kabelgebundene Verbindung mit der externen Empfangseinrichtung verzichtet werden. Hierdurch ergibt sich ein vereinfachter Aufbau. Zudem ermöglicht die drahtlose Übertragung auch eine Verbindung mit örtlich weiter entfernten externen Empfangseinrichtungen. Die Funkeinheit kann beispielsweise als Bluetooth-, WLAN- oder sonstige Einheit zur drahtlosen Übertragung von Signalen eingerichtet sein. Bei einer Ausgestaltung erfolgt die drahtlose Übertragung in verschlüsselter Form.

In weiterer Ausgestaltung der Erfindung weist die medizinische Vorrichtung zudem eine Befestigungseinrichtung auf, die zur lösbaren Befestigung der Vorrichtung an einem Außenumfang der Injektionsleitung eingerichtet ist. Die Befestigungseinrichtung erlaubt eine lösbare Befestigung wenigstens des Dehnungsmessstreifens und der Auswerteeinrichtung an der Injektionsleitung. Vorzugsweise und sofern vorhanden ist auch die Ausgabeeinrichtung mittels der Befestigungseinrichtung gemeinsam mit den weiteren Komponenten der medizinischen Vorrichtung an der Injektionsleitung lösbar befestigbar. Entsprechendes gilt sinngemäß, sofern alternativ oder zusätzlich die Übertragungseinrichtung vorhanden ist. Die Befestigungseinrichtung kann grundsätzlich auf jede für den genannten Zweck geeignete Weise gestaltet sein, beispielsweise zur form- und/oder kraftschlüssigen lösbaren Befestigung am Außenumfang der Injektionsleitung. Beispielsweise kann die Befestigungseinrichtung zum Ausbilden einer lösbaren Steck-, Klemm-, Rast- oder sonstigen lösbaren Fügeverbindung mit dem Außenumfang der Injektionsleitung eingerichtet sein.

In weiterer Ausgestaltung der Erfindung weist die Befestigungseinrichtung eine Hülse mit einem Innenumfang auf, der zum Ausbilden einer lösbaren Verbindung mit dem Außenumfang der Injektionsleitung eingerichtet ist, wobei der Dehnungsmessstreifen an dem Innenumfang der Hülse angeordnet ist. Zwecks lösbarer Befestigung an der Injektionsleitung kann die Hülse radial und/oder axial auf die Injektionsleitung aufgeschoben werden. Diese Ausgestaltung erlaubt eine besonders einfache lösbare Befestigung an der Injektionsleitung. Durch die Anordnung des Dehnungsmessstreifens am Innenumfang der Hülse ist gewährleistet, dass der Dehnungsmessstreifen in einer funktionsgerechten Art und Weise am Außenumfang der Injektionsleitung positioniert ist, um deren elastische Deformation und damit indirekt den Fluiddruck erfassen zu können.

Die Erfindung betrifft zudem ein medizinisches System mit einer offenbarungsgemäßen medizinischen Vorrichtung. Das erfindungsgemäße medizinische System umfasst zudem eine/die Injektionsleitung. Vorzugsweise ist das medizinische System zur Verwendung bei einer Regionalanästhesie vorgesehen und dementsprechend eingerichtet. Bei einer Ausgestaltung ist die medizinische Vorrichtung lösbar mit der Injektionsleitung verbunden oder lösbar verbindbar. Hierdurch kann die medizinische Vorrichtung mehrfach verwendet werden, wohingegen die Injektionsleitung als Einmalartikel gestaltet ist. Bei einer weiteren Ausgestaltung ist die medizinische Vorrichtung wenigstens teilweise mit der Injektionsleitung fest verbunden und/oder in diese integriert.

In weiterer Ausgestaltung der Erfindung ist die Injektionsleitung, insbesondere ein Katheter, zur Verwendung bei einer Regionalanästhesie. Solche Injektionsleitungen, im Speziellen Katheter, zeichnen sich durch eine - in Relation zu Injektionsleitungen für andere medizinische Anwendungen - geringe Drucknachgiebigkeit aus. Mit anderen Worten weist die Injektionsleitung bei dieser Ausgestaltung eine relativ geringe elastische Dehnung unter Einwirkung des zu überwachenden Fluiddrucks auf. Die medizinische Vorrichtung, im Speziellen der Dehnungsmessstreifen, ist derart eingerichtet, dass - trotz der geringen elastischen Dehnung - eine zuverlässige Erfassung des Fluiddruckes gewährleistet ist. Hierdurch wird eine verbesserte Regionalanästhesie ermöglicht.

In weiterer Ausgestaltung der Erfindung ist wenigstens der Dehnungsmessstreifen in die Injektionsleitung integriert, beispielsweise in einen Adapter der Injektionsleitung. Bei einer weiteren Ausgestaltung ist zudem die Auswerteeinrichtung, die Ausgabeeinrichtung und/oder die Übertragungseinrichtung in die Injektionsleitung integriert.

Die Erfindung betrifft zudem ein Verfahren zum Überwachen eines Fluiddrucks in einer Injektionsleitung. Das erfindungsgemäße Verfahren weist Schritte auf: Erfassen des Fluiddrucks, wobei der Fluiddruck mittelbar durch Erfassen einer durch den Fluiddruck verursachten elastischen Deformation der Injektionsleitung erfasst wird, und wobei die elastische Deformation mittels eines Dehnungsmessstreifens erfasst wird, der ein Messsignal erzeugt, das den Fluiddruck repräsentiert; Vergleichen des Messsignals mit wenigstens einem vorgegebenen Schwellenwert und Erzeugen wenigstens eines Überwachungssignals in Abhängigkeit des Vergleichs; Ausgeben des wenigstens einen Überwachungssignals in einer für einen Benutzer wahrnehmbaren Form; und/oder Übertragen des wenigstens einen Überwachungssignals an eine externe Empfangseinrichtung. Zu den mit dem erfindungsgemäßen Verfahren einhergehenden Vorteilen wird zur Vermeidung von Wiederholungen auf die Offenbarung zu der erfindungsgemäßen medizinischen Vorrichtung verwiesen und ausdrücklich Bezug genommen. Das dort Offenbarte gilt mutatis mutandis auch für das erfindungsgemäße Verfahren. Ausgestaltungen des erfindungsgemäßen Verfahrens ergeben sich unmittelbar und eindeutig aus der Offenbarung der Ausgestaltungen der erfindungsgemäßen medizinischen Vorrichtung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.

Es zeigen:
- Fig. 1: in schematischer Blockansicht eine Ausführungsform einer erfindungsgemäßen medizinischen Vorrichtung zum Überwachen eines Fluiddrucks in einer Injektionsleitung,
- Fig. 2: in schematisch vereinfachter Schnittansicht eine Ausführungsform einer erfindungsgemäßen medizinischen Vorrichtung mit optischer Signalisierung des überwachten Fluiddrucks,
- Fig. 3: eine weitere schematische Blockansicht zur Verdeutlichung weiterer Merkmale der Ausführungsform nach Fig. 2,
- Fig. 4: in schematisch vereinfachter Schnittansicht eine Ausführungsform einer erfindungsgemäßen medizinischen Vorrichtung mit akustischer Signalisierung des überwachten Fluiddrucks,
- Fig. 5: ein schematisch vereinfachtes Diagramm zur Verdeutlichung weiterer Merkmale der medizinischen Vorrichtung nach Fig. 4,
- Fig. 6: in schematischer Schnittansicht eine Ausführungsform einer erfindungsgemäßen medizinischen Vorrichtung mit drahtloser Übertragung eines den Fluiddruck repräsentierenden Überwachungssignals, und
- Fig. 7: in schematischer Blockansicht eine Ausführungsform eines erfindungsgemäßen Verfahrens.

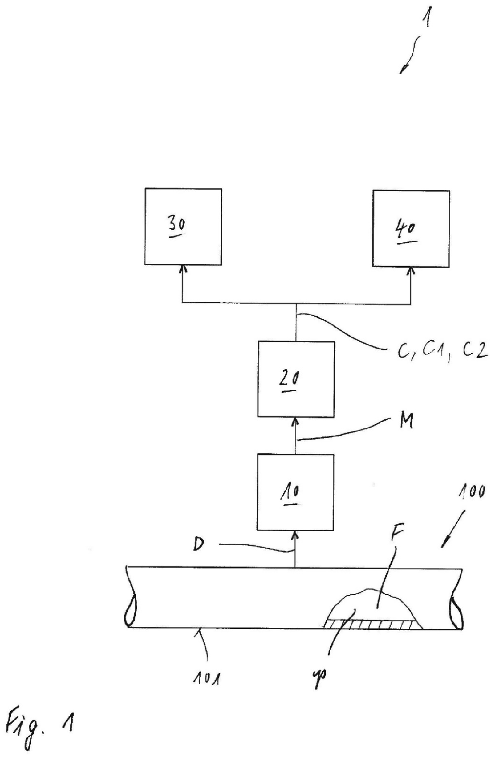

Gemäß Fig. 1 ist eine medizinische Vorrichtung 1 zum Überwachen eines Fluiddrucks p eines medizinischen Fluids F in einer Injektionsleitung 100 vorgesehen. Bei dem medizinischen Fluid F handelt es sich vorliegend um ein Anästhetikum. Die Injektionsleitung 100 ist in Fig. 1 axial beidseits abgeschnitten dargestellt und wird zur Verabreichung des Anästhetikums bei einer peripheren Lokalanästhesie verwendet.

Die medizinische Vorrichtung 1 weist einen Dehnungsmessstreifen 10 und eine Auswerteeinrichtung 20 auf. Zudem weist die medizinische Vorrichtung 1 eine Ausgabeeinrichtung 30 sowie alternativ oder zusätzlich eine Übertragungseinrichtung 40 auf.

Der Dehnungsmessstreifen 10, die Auswerteeinrichtung 20, die Ausgabeeinrichtung 30 und die Übertragungseinrichtung 40 sind in Fig. 1 schematisch stark vereinfacht als Funktionsblöcke dargestellt.

Der Dehnungsmessstreifen 10 ist zum Erfassen einer durch den Fluiddruck p verursachten elastischen Deformation D der Injektionsleitung 100 und zum Ausgeben eines Messsignals M eingerichtet. Das Messsignal M repräsentiert die erfasste elastische Deformation D und damit indirekt auch den Fluiddruck p.

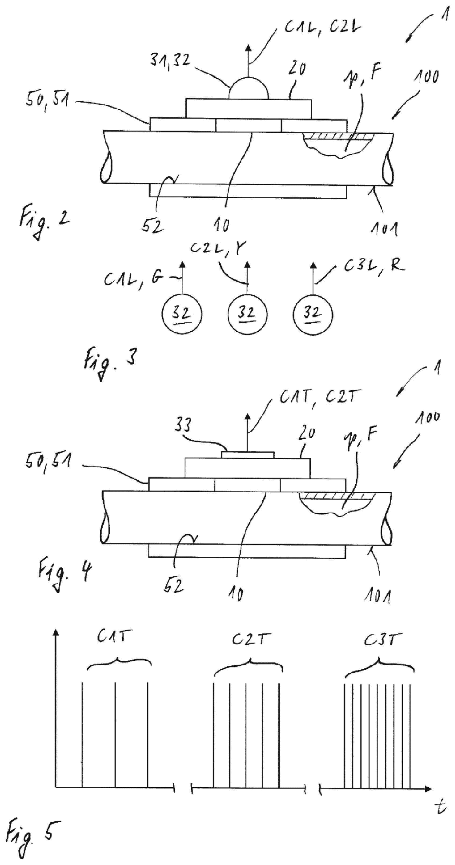

Bei einer Ausgestaltung ist der Dehnungsmessstreifen Bestandteil der Injektionsleitung. Da die Injektionsleitung in der Regel nach einmaliger Verwendung entsorgt wird, ist bei solchen Ausgestaltungen eine mehrfache Verwendung des Dehnungsmessstreifens nicht vorgesehen. Bei weiteren Ausgestaltungen, wie sie beispielhaft nachfolgend noch anhand der Fig. 2, 4 und 6 erläutert werden, ist der Dehnungsmessstreifen zur lösbaren Befestigung an der Injektionsleitung eingerichtet. Hierdurch kann der Dehnungsmessstreifen und damit auch die gesamte medizinische Vorrichtung mehrfach verwendet werden.

Die Auswerteeinrichtung 20 ist mit dem Dehnungsmessstreifen 10 verbunden und zum Vergleichen des erfassten Messsignals M mit einem vorgegebenen Schwellenwert eingerichtet. Weiter ist die Auswerteeinrichtung 20 zum Erzeugen wenigstens eines Überwachungssignals C in Abhängigkeit des Vergleichs eingerichtet.

Der besagte Schwellenwert kann beispielsweise als fester Wert in einer Speichereinheit der Auswerteeinrichtung hinterlegt sein. Alternativ oder zusätzlich ist eine Vorgabe des Schwellenwerts mittels einer Eingabeeinrichtung denkbar und möglich.

Das Vergleichen des Messsignals M mit dem Schwellenwert umfasst beispielsweise eine Differenzbildung, Subtraktion oder dergleichen. Das Überwachungssignal C wird in Abhängigkeit des besagten Vergleichs erzeugt.

Bei einer Ausgestaltung wird das Überwachungssignal C beispielsweise nur dann erzeugt, wenn das Messsignal M den Schwellenwert überschreitet. In diesem Fall repräsentiert der Schwellenwert einen idealerweise nicht zu überschreitenden maximalen Fluiddruck des Fluids F.

Die Ausgabeeinrichtung 30 ist mit der Auswerteeinrichtung 20 verbunden und zum Ausgeben des wenigstens einen Überwachungssignals C in einer für einen Benutzer wahrnehmbaren Form eingerichtet. Bei der für den Benutzer wahrnehmbaren Form der Signalausgabe handelt es sich bei einer Ausgestaltung um eine optische Ausgabe. Alternativ oder zusätzlich kann eine akustische Ausgabe vorgesehen sein. Die Ausgabeeinrichtung ist in beiden Fällen entsprechend eingerichtet. Näheres hierzu wird nachfolgend noch unter Bezugnahme auf die Ausführungsformen nach den Fig. 2 und 3 sowie 4 und 5 erläutert.

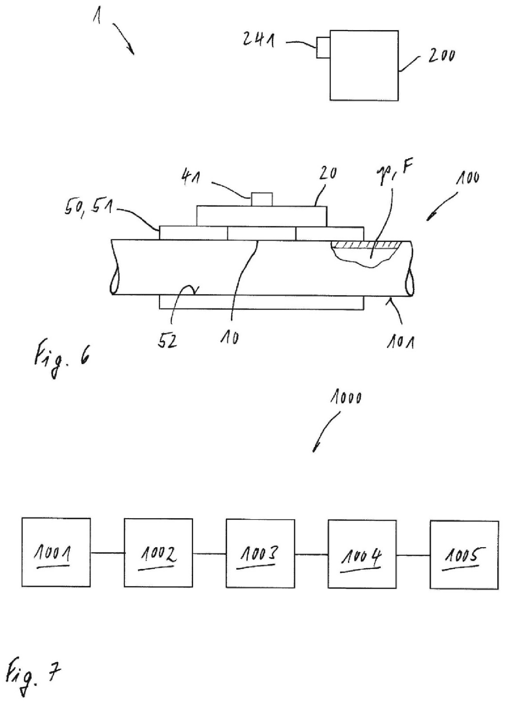

Die alternativ oder zusätzlich zu der Ausgabeeinrichtung 30 vorhandene Übertragungseinrichtung 40 ist mit der Auswerteeinrichtung 20 verbunden und zum Übertragen des wenigstens einen Überwachungssignals C an eine externe Empfangseinrichtung 200 eingerichtet (siehe Fig. 6). Bei einer Ausgestaltung ist die Übertragungseinrichtung zum drahtlosen Übertragen des wenigstens einen Überwachungssignals eingerichtet. Alternativ oder zusätzlich kann eine drahtgebundene Übertragung vorgesehen sein. Das auf diese Weise übertragene wenigstens eine Überwachungssignal C kann mittels der externen Empfangseinrichtung ausgegeben, weiterverarbeitet, dokumentiert und/oder auf sonstige Weise weiterbehandelt werden. Die externe Empfangseinrichtung ist keine Komponente der medizinischen Vorrichtung 1 nach Fig. 1. Bei einer in den Figuren nicht dargestellten Ausführungsform ist die externe Empfangseinrichtung eine Komponente einer medizinischen Anordnung, welche auch die medizinische Vorrichtung umfasst.

Bei der Ausführungsform nach Fig. 1 ist die Auswerteeinrichtung 20 weiter dazu eingerichtet, in Abhängigkeit des Vergleichs (des Messsignals M mit dem Schwellenwert) wenigstens ein erstes Überwachungssignal C1 und ein zweites Überwachungssignal C2 zu erzeugen.

Das erste Überwachungssignal C1 wird erzeugt, falls der Schwellenwert unterschritten ist. Das unterschiedliche zweite Überwachungssignal C2 wird erzeugt, falls der Schwellenwert überschritten ist. Dementsprechend ist die Ausgabeeinrichtung 30 zum Ausgeben wenigstens des ersten Überwachungssignals C1 und des unterschiedlichen zweiten Überwachungssignals C2 eingerichtet. Entsprechendes gilt sinngemäß für die weitergehende Einrichtung der Übertragungseinrichtung 40. Mit anderen Worten: Die Übertragungseinrichtung 40 erlaubt bei der Ausführungsform nach Fig. 1 eine Übertragung der unterschiedlichen Überwachungssignale C1, C2.

Durch das Erzeugen sowie Ausgeben und/oder Übertragen der wenigstens zwei unterschiedlichen Überwachungssignale C1, C2 kann auf einfache Weise überwacht werden, ob der Fluiddruck p niedrig (Schwellenwert unterschritten, erstes Überwachungssignal C1) und damit unkritisch oder hoch (Schwellenwert überschritten, zweites Überwachungssignal C2) und damit potenziell kritisch ist. Es versteht sich, dass die Auswerteeinrichtung in Abhängigkeit des Vergleichs auch zum Erzeugen noch weiterer unterschiedlicher Überwachungssignale, beispielsweise eines dritten Überwachungssignals, eingerichtet sein kann. Zudem ist es denkbar und möglich, dass der Vergleich einen weiteren oder mehrere weitere Schwellenwerte umfasst.

Die medizinische Vorrichtung 1 bildet zusammen mit der Injektionsleitung 100 ein medizinisches System.

Bei der Ausführungsform nach Fig. 2 ist eine optische Überwachung des Fluiddrucks p vorgesehen. Zu diesem Zweck weist die Ausgabeeinrichtung 30 wenigstens ein Leuchtmittel 31 auf. Das Leuchtmittel 31 ist zum Ausgeben wenigstens des ersten Überwachungssignals C1 und des zweiten Überwachungssignals C2 in Form unterschiedlicher Lichtsignale C1L, C2L eingerichtet. Die unterschiedlichen Lichtsignale C1L, C2L können sich grundsätzlich im Hinblick auf ihre jeweilige Lichtfarbe und/oder Signaldauer unterscheiden.

Bei der Ausführungsform nach Fig. 2 ist das wenigstens eine Leuchtmittel eine RGB-LED 32, die zur Ausgabe unterschiedlich farbiger Lichtsignale eingerichtet ist.

Wie anhand Fig. 3 verdeutlicht ist, ist bei der Ausführungsform nach Fig. 2 vorgesehen, dass drei unterschiedliche Überwachungssignale mittels der Auswerteeinrichtung 20 erzeugt und in Form eines ersten Lichtsignals C1L mit grüner Lichtfarbe G, eines unterschiedlichen zweiten Lichtsignals C2L mit gelber Lichtfarbe Y und eines weiteren unterschiedlichen Lichtsignals C3L mit roter Lichtfarbe R mittels der RGB-LED 32 ausgegeben werden. Das erste Lichtsignal C1L mit grüner Lichtfarbe G signalisiert dabei einen niedrigen Druckbereich des Fluiddrucks p. Das zweite Lichtsignal C2L mit gelber Lichtfarbe Y signalisiert einen mittleren Druckbereich des Fluiddrucks p. Das dritte Lichtsignal C3L mit roter Lichtfarbe R signalisiert einen hohen Druckbereich des Fluiddrucks p.

Anhand der unterschiedlichen Lichtfarben G, Y, R der Lichtsignale C1L, C2L, C3L kann der Benutzer auf besonders einfache und zuverlässige Weise erkennen, ob der Fluiddruck p unkritisch (grün), möglicherweise kritisch (gelb) oder kritisch (rot) ist. Mit anderen Worten: Die Ausführungsform nach den Fig. 2 und 3 signalisiert den Druckbereich des Fluiddrucks p nach Art einer Ampelanzeige.

Bei der Ausführungsform nach den Fig. 2 und 3 weist die medizinische Vorrichtung 1 zudem eine Befestigungseinrichtung 50 auf. Die Befestigungseinrichtung 50 ist zur lösbaren Befestigung der medizinischen Vorrichtung 1 an einem Außenumfang 101 der Injektionsleitung 100 eingerichtet.

Dabei sind sämtliche weiteren Komponenten der medizinischen Vorrichtung 1 unmittelbar oder mittelbar an der Befestigungseinrichtung 50 angebracht und/oder gelagert, so insbesondere der Dehnungsmessstreifen 10, die Auswerteeinrichtung 20 und die Ausgabeeinrichtung 30 in Form des Leuchtmittels 31, im Speziellen in Form der RGB-LED 32.

Bei der in Fig. 2 gezeigten Ausführungsform weist die Befestigungseinrichtung 50 eine Hülse 51 mit einem Innenumfang 52 auf, der zum Ausbilden einer lösbaren Verbindung mit dem Außenumfang 101 der Injektionsleitung 100 eingerichtet ist. Zum Ausbilden der besagten lösbaren Verbindung kann die Hülse 51 in radialer Richtung und/oder axialer Richtung auf den Außenumfang 101 der Injektionsleitung 100 aufgeschoben werden. Bei einer Ausgestaltung wird hierbei eine Klemmverbindung ausgebildet. Alternativ oder zusätzlich kann es sich bei der lösbaren Verbindung zwischen Hülse 51 und Injektionsleitung 100 um eine Rast-, Steck- oder sonstige für den vorliegenden Zweck geeignete lösbare Fügeverbindung handeln.

Bei der Ausführungsform nach Fig. 4 weist die medizinische Vorrichtung 1 anstelle des wenigstens einen Leuchtmittels 31 einen Lautsprecher 33 zur akustischen Signalisierung des überwachten Fluiddrucks p auf. Im Übrigen ist der Aufbau der medizinischen Vorrichtung 1 nach Fig. 4 identisch mit dem der medizinischen Vorrichtung 1 nach Fig. 2, so dass zur Vermeidung von Wiederholungen auf die dortige Offenbarung verwiesen und Bezug genommen wird. Sofern nichts anderes beschrieben ist, gilt für die Ausführungsform nach Fig. 4 das bereits zu der Ausführungsform nach Fig. 2 Offenbarte sinngemäß.

Der Lautsprecher 33 ist zum Ausgeben wenigstens des ersten Überwachungssignals C1 und des zweiten Überwachungssignals C2 in Form unterschiedlicher Tonsignale C1T, C2T eingerichtet, wobei die unterschiedlichen Tonsignale C1T, C2T sich im Hinblick auf ihre jeweilige Tonhöhe, Tonfolge und/oder Signaldauer unterscheiden.

Anhand Fig. 5 sind drei unterschiedliche Tonsignale C1T, C2T, C3T verdeutlicht, die auch als erstes Tonsignal C1T, zweites Tonsignal C2T und drittes Tonsignal C3T bezeichnet werden können. Das erste Tonsignal C1T signalisiert vorliegend einen niedrigen Druckbereich, das zweite Tonsignal C2T einen mittleren Druckbereich und das dritte Tonsignal C3T einen hohen Druckbereich des Fluiddrucks p. Alternativ zu unterschiedlichen Tonsignalen kann auch eine stufenloser An- und/oder Abstieg vorgesehen sein.

Die unterschiedlichen Tonsignale C1T, C2T, C3T weisen dabei vorliegend jeweils eine identische Tonhöhe und zudem eine insgesamt identische Signaldauer auf. Allerdings unterscheiden die unterschiedlichen Tonsignale C1T, C2T, C3T sich im Hinblick auf ihre Tonfolge. So umfasst das erste Tonsignal C1T vorliegend drei Einzeltöne, das zweite Tonsignal C2T umfasst bei identischer gesamter Signaldauer fünf Einzeltöne und das dritte Tonsignal C3T umfasst bei identischer gesamter Signaldauer neun Einzeltöne. Die genannten Anzahlen an Einzeltönen sind als rein exemplarisch zu verstehen. Zudem versteht sich, dass die Tonsignale C1T, C2T, C3T wenigstens einmalig oder auch mehrfach sowie fortdauernd wiederholt werden können, solange der Fluiddruck p in dem besagten Druckbereich anliegt.

Bei der Ausgestaltung nach Fig. 6 weist die medizinische Vorrichtung anstelle der/einer Ausgabeeinrichtung 30 die bereits erwähnte Übertragungseinrichtung 40 auf, die in diesem Fall mit einer Funkeinheit 41 versehen ist, die eine drahtlose Übertragung des wenigstens einen Überwachungssignals C an die externe Empfangseinrichtung 200 erlaubt. Die externe Empfangseinrichtung 200 ist mit einer komplementären Funkeinheit 241 ausgestattet, die ein Empfangen der mittels der Funkeinheit 41 gesendeten Signale erlaubt.

In Fig. 7 ist schematisch ein Verfahren 1000 zum Überwachen des Fluiddrucks p in der besagten Injektionsleitung 100 gezeigt. Die anhand der Fig. 1 bis 6 verdeutlichten medizinischen Vorrichtungen 1 sind jeweils zum Ausführen des Verfahrens 1000 nach Fig. 7 eingerichtet.

Das Verfahren 1000 sieht Schritte 1001 bis 1005 vor, nämlich: Erfassen 1001 des Fluiddrucks p, wobei der Fluiddruck p mittelbar durch Erfassen einer durch den Fluiddruck p verursachten elastischen Deformation D der Injektionsleitung 100 erfasst wird, und wobei die elastische Deformation D mittels eines Dehnungsmessstreifens 10 (siehe Fig. 1, 2, 4, 6) erfasst wird, der ein Messsignal M erzeugt, das den Fluiddruck p repräsentiert; Vergleichen 1002 des Messsignals M mit wenigstens einem vorgegebenen Schwellenwert und Erzeugen 1003 wenigstens eines Überwachungssignals C in Abhängigkeit des Vergleichs; Ausgeben 1004 des wenigstens einen Überwachungssignals C in einer für einen Benutzer wahrnehmbaren Form; alternativ oder zusätzlich zu dem Schritt 1004 ist ein Übertragen 1005 des wenigstens einen Überwachungssignals C an die externe Empfangseinrichtung 200 vorgesehen.

## Patentansprüche

1. Medizinische Vorrichtung (1) zum Überwachen eines Fluiddrucks (p) in einer Injektionsleitung (100), aufweisend
einen Dehnungsmessstreifen (10), der zum Erfassen einer durch den Fluiddruck (p) verursachten elastischen Deformation (D) der Injektionsleitung (100) und zum Ausgeben eines Messsignals (M) eingerichtet ist, das den erfassten Fluiddruck (p) repräsentiert,
eine Auswerteeinrichtung (20), die mit dem Dehnungsmessstreifen (10) verbunden ist, wobei die Auswerteeinrichtung (20) eingerichtet ist zum Vergleichen des Messsignals (M) mit wenigstens einem vorgegebenen Schwellenwert, und zum Erzeugen wenigstens eines Überwachungssignals (C) in Abhängigkeit des Vergleichs,
eine Ausgabeeinrichtung (30) und/oder eine Übertragungseinrichtung (40), wobei die Ausgabeeinrichtung (30) mit der Auswerteeinrichtung (20) verbunden und zum Ausgeben des wenigstens einen Überwachungssignals (C) in einer für einen Benutzer wahrnehmbaren Form eingerichtet ist,
wobei die Übertragungseinrichtung (40) mit der Auswerteeinrichtung (20) verbunden und zum Übertragen des wenigstens einen Überwachungssignals (C) an eine externe Empfangseinrichtung (200) eingerichtet ist.

2. Medizinische Vorrichtung (1) nach Anspruch 1, wobei die Auswerteeinrichtung (20) dazu eingerichtet ist, in Abhängigkeit des Vergleichs wenigstens ein erstes Überwachungssignal (C1) zu erzeugen, falls der Schwellenwert unterschritten ist, und ein unterschiedliches zweites Überwachungssignal (C2) zu erzeugen, falls der Schwellenwert überschritten ist, und wobei die Ausgabeeinrichtung (30) zum Ausgeben des ersten Überwachungssignals (C1) und des zweiten Überwachungssignals (C2) eingerichtet ist.

3. Medizinische Vorrichtung (1) nach Anspruch 1 oder 2, wobei die Ausgabeeinrichtung (30) wenigstens ein Leuchtmittel (31) aufweist, das zum Ausgeben wenigstens des ersten Überwachungssignals (C1) und des zweiten Überwachungssignals (C2) in Form unterschiedlicher Lichtsignale (C1L, C2L) eingerichtet ist, wobei die unterschiedlichen Lichtsignale (C1L, C2L) sich im Hinblick auf ihre jeweilige Lichtfarbe (G, Y, R) und/oder Signaldauer unterscheiden.

4. Medizinische Vorrichtung (1) nach Anspruch 3, wobei das wenigstens eine Leuchtmittel (31) eine RGB-LED (32) ist, die zur Ausgabe grüner, gelber und roter Lichtsignale (C1L, G; C2L, Y; C3L, R) eingerichtet ist, die einen niedrigen, mittleren bzw. hohen Fluiddruck repräsentieren.

5. Medizinische Vorrichtung (1) nach einem der Ansprüche 2 bis 4, wobei die Ausgabeeinrichtung (30) einen Lautsprecher (33) aufweist, der zum Ausgeben wenigstens des ersten Überwachungssignals (C1) und des zweiten Überwachungssignals (C2) in Form unterschiedlicher Tonsignale (C1T, C2T) eingerichtet ist, wobei die unterschiedlichen Tonsignale (C1T, C2T) sich im Hinblick auf ihre jeweilige Tonhöhe, Tonfolge und/oder Signaldauer unterscheiden.

6. Medizinische Vorrichtung (1) nach einem der vorhergehenden Ansprüche, wobei die Übertragungseinrichtung (40) eine Funkeinheit (41) aufweist, die zur drahtlosen Übertragung des wenigstens einen Überwachungssignals (C) eingerichtet ist.

7. Medizinische Vorrichtung (1) nach einem der vorhergehenden Ansprüche, weiter aufweisend eine Befestigungseinrichtung (50), die zur lösbaren Befestigung der Vorrichtung (1) an einem Außenumfang (101) der Injektionsleitung (100) eingerichtet ist.

8. Medizinische Vorrichtung (1) nach Anspruch 7, wobei die Befestigungseinrichtung (50) eine Hülse (51) mit einem Innenumfang (52) aufweist, der zum Ausbilden einer lösbaren Verbindung mit dem Außenumfang (101) der Injektionsleitung (100) eingerichtet ist, und wobei der Dehnungsmessstreifen (10) an dem Innenumfang (52) der Hülse (51) angeordnet ist.

9. Medizinisches System mit einer medizinischen Vorrichtung (1) nach einem der vorhergehenden Ansprüche und mit einer/der Injektionsleitung (100).

10. Medizinisches System nach Anspruch 9, wobei die Injektionsleitung (100), insbesondere als Katheter, zur Verwendung bei einer Regionalanästhesie eingerichtet ist.

11. Medizinisches System nach Anspruch 9 oder 10, wobei wenigstens der Dehnungsmessstreifen (10) in die Injektionsleitung (100) integriert ist, insbesondere wobei weiter die Auswerteeinrichtung (20) und/oder die Ausgabeeinrichtung (30) und/oder die Übertragungseinrichtung (40) in die Injektionsleitung (100) integriert ist.

12. Verfahren (1000) zum Überwachen eines Fluiddrucks (p) in einer Injektionsleitung (100), aufweisend Schritte:
Erfassen (1001) des Fluiddrucks (p), wobei der Fluiddruck (p) mittelbar durch Erfassen einer durch den Fluiddruck (p) verursachten elastischen Deformation (D) der Injektionsleitung (100) erfasst wird, und wobei die elastische Deformation (D) mittels eines Dehnungsmessstreifens (10) erfasst wird, der ein Messsignal (M) erzeugt, das den Fluiddruck (p) repräsentiert;
Vergleichen (1002) des Messsignals (M) mit wenigstens einem vorgegebenen Schwellenwert und Erzeugen (1003) wenigstens eines Überwachungssignals (C) in Abhängigkeit des Vergleichs;
Ausgeben (1004) des wenigstens einen Überwachungssignals (C) in einer für einen Benutzer wahrnehmbaren Form und/oder Übertragen (1005) des wenigstens einen Überwachungssignals (C) an eine externe Empfangseinrichtung (200).
